# EUROPÄISCHE PATENTSCHRIFT

(11) **EP 1 848 446 B1**
(45) Veröffentlichungstag und Bekanntmachung des Hinweises auf die Patenterteilung: **14.10.2009**
(21) Anmeldenummer: 06707742.0
(22) Anmeldetag: 18.01.2006
(51) Int. Cl.: A61K 33/26, A61K 47/26, A61P 25/28, A61P 37/02

(54) **VERWENDUNG VON EISEN(III)-KOMPLEXVERBINDUNGEN**
USE OF IRON(III) COMPLEX COMPOUNDS
UTILISATION DE COMPOSES COMPLEXES DE FER (III)

(30) Priorität: 09.02.2005 EP 05100907
(43) Veröffentlichungstag der Anmeldung: 31.10.2007
(73) Patentinhaber: VIFOR (INTERNATIONAL) AG, 9001 St. Gallen (CH)
(72) Erfinder: TANNER-BAUMGARTNER, Jessica, CH-9010 St. Gallen (CH); CHANDRA, Ranjeet, Etobicoke, Ontario M9B6L9 (CA); GEISSER, Peter, CH-9014 St. Gallen (CH)
(74) Vertreter: Gille Hrabal Struck Neidlein Prop Roos
(86) Internationale Anmeldenummer: PCT/EP2006/050276
(87) Internationale Veröffentlichungsnummer: WO 2006/084782

(56) Entgegenhaltungen:
- WO-A-02/46241
- WO-A-95/35113
- WO-A-20/04037865
- KOLB E: "[Relation of iron supply and iron metabolism to infectious diseases and parasitoses and the competence of the immune system]" ZEITSCHRIFT FUR DIE GESAMTE INNERE MEDIZIN UND IHRE GRENZGEBIETE ... 1 NOV 1988, Bd. 43, Nr. 21, 1. November 1988 (1988-11-01), Seiten 597-601, XP009050945 ISSN: 0044-2542
- ANDREWS N C ET AL: "Disorders of red cell iron during infancy and childhood" INTERNATIONAL JOURNAL OF PEDIATRIC HEMATOLOGY/ONCOLOGY 1997 UNITED KINGDOM, Bd. 4, Nr. 2, 1997, Seiten 171-180, XP009050932 ISSN: 1070-2903

## Beschreibung

Die vorliegenden Erfindung betrifft neue therapeutische Anwendungen von Eisen(III)-Komplexverbindungen mit Kohlehydraten oder Derivaten davon, insbesondere mit Dextrinen oder Oxidationsprodukten von Dextrinen, insbesondere zur Herstellung von Arzneimitteln zur Verbesserung der Immunabwehr und/oder der Hirnleistung bei Patienten ohne Eisenmangel oder Eisenmangelanämie.

Eisenmangel ist der häufigste Spurenelementmangel weltweit. Nicht nur Kinder, vom Säugling bis zum Heranwachsenden, in den Entwicklungsländern sind betroffen, sondern er wird auch bei einer signifikanten Anzahl von Kindern in den industrialisierten, reichen Ländern beobachtet. Z.B. zeigen 28 % der Teenager in Kanada Anzeichen von Eisenmangel.

Aus der WO 95/35113 ist die Verwendung von Eisen(III)-oxid als Wirkstoff zur Behandlung von Immunschwächerkrankungen insbesondere AIDS bekannt.

Aus der DE 1 467980 sind therapeutisch verwendbare Eiseninjektionspräparate und Verfahren zu Ihrer Herstellung bekannt.

Aus der US 3076798 sind Verfahren zur Herstellung von Eisen(III)-Polymaltose-Komplexverbindungen bekannt, die zur parenteralen Verabreichung geeignet sind.

Aus der WO 2004037865 ist die Verwendung von Eisen-Kohlenhydrat-Komplexen zur Behandlung oder Prophylaxe von Eisenmangelzuständen bekannt.

Aus der WO 03/087164 sind Eisen-Komplexverbindungen mit hydrierten Dextrinen zur Behandlung oder Prophylaxe von Eisenmangelzuständen bekannt.

Aus der WO 02/46241 sind Eisen(III)-Pullulan-Komplexverbindungen und ihre Verwendung zur Behandlung oder Prophylaxe von Eisenmangelzustönden bekannt.

Aus Baumgartner "New Aspects of Iron Therapy", Second Ferrum Meeting, Lissabon 1994 ist bekannt, dass sowohl die Aktivität als auch die kognitive Leistung der linken Hirnhemisphäre vom Eisenstatus eines Individuums abhängen. Darin erwähnt Baumgartner eine Studie von Walter, wonach eine Eisenanämie signifikant zu einer eingeschränkten mentalen und psychomotorischen Entwicklung bei Kindern führt. Die in der Studie verwendete Verbindung war Eisensulfat. Nach 75 Tagen der Verabreichung von Eisensulfat fand Walter jedoch keine Verbesserung der mentalen und psychomotorischen Entwicklung.

Andere bei Baumgartner erwähnte Studien zeigen eine signifikante Verbesserung der kognitiven Leistung bei einer Eisentherapie.

Weiterhin stellt Baumgartner klinische Studien vor, die den Einfluß des Eisenstatus auf die immunologischen Funktionen reflektieren.

Eisensulfat ist dafür bekannt, dass es relativ häufig unangenehme dosisabhängige Nebenreaktionen, wie gastrointestinale Störungen oder eine Verfärbung der Zähne hervorruft. Eisen aus Eisensalz-Verbindungen unterliegt der passiven Diffusion freier Eisenionen. Das Eisen kann in den Kreislauf eintreten und dadurch Nebenreaktionen oder eine Eisenvergiftung hervorrufen. Dementsprechend ist auch der LD50-Wert bei weißen Mäusen mit 230 mg Eisen/kg relativ niedrig. Die Verwendung von Eisensalz-Verbindungen ist daher insbesondere bei der Behandlung von Kindern, bei denen Eisen besonders wichtig für die Entwicklung des Gehirns und des Immunsystems ist, nachteilig.

Die in der Veröffentlichung von Baumgartner ebenfalls erwähnte Studie Tucker "Iron status and brain function: serum ferritin levels associated with asymmetries of cortical electrophysiology and cognitive performance" (Am. J. Clin. Nutr. 1984; 39: 105-113) zeigt, dass die Hirnleistung proportional zum Ferritinspiegel ist.

Oski "Effect of Iron Therapy on Behavior Performance in Nonannemic, Iron-Deficient Infants", PEDIATRICS 1983; Band 71; 877-880 verwendet Eisen-Dextran. Die parenterale Verwendung von Eisen-Dextran ist nachteilig, weil ein Dextran-induzierter anaphylaktischer Schock auftreten kann.

WO 2004 / 037865 der Anmelderin offenbart die Verwendung der in der vorliegenden Anwendung verwendeten Eisen (III) - Komplexverbindungen zur Herstellung eines Medikaments zur Behandlung von Eisenmangel und Eisenmangelanämie und weist auf die bessere Verträglichkeit dieser Komplexe hin.

WO 02 / 46241 offenbart die Verwendung von Eisen (III) - Pullulan - Komplexen zur Herstellung eines Medikaments zur Behandlung von Eisenmangel und Eisenmangelanämie.

WO 95/ 35113 offenbart die Wirksamkeit von Eisen (III) - Verbindungen zur Behandlung von Immunschwäche. Es wird eine antivirale in-vitro-Wirksamkeit gegenüber HIV - Stämmen beschrieben.

Zeitschrift für die Gesamte Innere Medizin und ihre Grenzgebiete, 1988, Band 43, Nr. 21 , S. 597-601 ist ein wissenschaftlicher Übersichtsartikel über die Beziehungen zwischen Eisenversorgung und Eisenstoffwechsel bei Infektionskrankheiten und Parasitosen sowie zur Leistungsfähigkeit des Immunsystems. Es wird insbesondere offenbart, dass die intramuskuläre Verabreichung bestimmter organischer Eisenverbindungen auf die Abwehr von Infektionen ungünstige Wirkungen hat.

International Journal of Pediatric Hematology / Oncology, Band 4, Seite 171 - 180 ist ein wissenschaftlicher Übersichtsartikel über Eisenerkrankungen in roten Blutzellen während Säuglingszeit und Kindheit. Dort werden in allgemeiner Form die Folgen von Eisenmangelanämien dargestellt. Eisen (III) - Komplexverbindungen werden nicht im Besonderen diskutiert.

Die Erfinder stellten sich daher die Aufgabe, gut verträgliche Eisenverbindungen zu finden, die geeignet sind, die Hirnleistung und die Immunabwehr insbesondere bei Kindern, einschließlich Säuglingen und Heranwachsenden zu verbessern.

In einer Studie konnten sie nachweisen, dass Eisen(III)-Komplexverbindungen mit Kohlehydraten, insbesondere mit Polymaltose (Maltodextrin) besonders verträglich sind, eine hohe Patienten-Compliance besitzen und eine signifikante Verbesserung der Immunabwehr und/oder der Hirnleistung bewirken. Überraschend war dabei auch, dass eine frühe signifikante Verbesserung der Immunabwehr und/oder der Hirnleistung gefunden wird, obwohl Eisen(III)-Polymaltose-Komplex-Verbindungen nur zu einer langsamen Erhöhung des Ferritinspiegels führen. Auf der Basis dieses Ergebnisses stellten sie die vorliegende Erfindung fertig. Gegenstand der Erfindung ist daher die Verwendung von Eisen(III)-Komplexverbindungen mit Kohlehydraten zur Herstellung eines Arzneimittels zur Verbesserung der Immunabwehr und/oder der Hirnleistung nach Anspruch 1.

Die Verbesserung der Immunabwehr im Sinne der Erfindung bedeutet eine signifikante Verbesserung der Immunantworten, wie sie sich beispielsweise in einer signifikanten Verbesserung der Lymphozytenantwort gegenüber Phytohämagglutlnin (PHA) unter Verwendung der MTT-Methode, in einer Verbesserung im Nitroblau-Tetrazoliumtest (MBT) unter Verwendung von Neutrophilen, in eine Verbesserung der bakteriziden Kapazität von Neutrophilen (PCA) gemessen durch das tubidimetrische Verfahren, in einer Verbesserung der monoklonalen Antikörper, beispielsweise CD3, CD4, CD8 und CD56, ausgezählt beispielsweise mit einem BD-Fließzytometer mit einfacher Färbemethode und/oder in der Antikörperantwort gegenüber Masern, H-Influenza und Tetanus zeigt.

Eine Verbesserung der Hirnleistung im Sinne der Erfindung schließt insbesondere eine Verbesserung der kognitiven Funktionen und des emotionalen Verhaltens ein und drückt sich beispielsweise aus in einer Verbesserung im Kurzzeitgedächtnistests (STM), im Langzeitgedächtnistest (LTM), im Test der progressiven Matrices nach Raven, in der Welschers Erwachsenenintelligenzskala (WAIS) und/oder im emotionalen Koeffizienten (Baron EQ-i, YV-Test; Jugendversion) aus.

Erfindungsgemäß anwendbare Eisen(III)-Komplexverbindungen mit Kohlehydraten schließen bevorzugt diejenigen ein, worin Kohlenhydrate aus der Gruppe ausgewählt werden, die aus Dextranen, Dextrinen sowie Pullulan, und Oligomeren davon besteht, Besonders bevorzugt sind Eisen(III)-Komplexverbindungen mit Dextrinen oder Oxidationsprodukten davon. Beispiele der Herstellung der erfindungsgemäßen Eisen(III)-Komplexverbindungen finden sich beispielsweise in den eingangs erwähnten Patentschriften DE 14679800, WO 04037865 A1, US 3076798, WO 03/087164 sowie WO 02/46241, deren Offenbarungsgehalt insbesondere hinsichtlich der Herstellverfahren hier vollumfänglich eingeschlossen sein soll. Der Begriff der erfindungsgemäß bevorzugt verwendeten "Dextrine" ist eine Sammelbezeichnung für verschiedene niedere und höhere Polymere aus D-Glucose-Einheiten, die bei unvollständiger Hydrolyse von Stärke entstehen. Sie können ferner durch Polymerisation von Zuckern hergestellt werden (z.B. WO02083739 A2, US20030044513 A1, US 3766165). Zu den Dextrinen gehören die Maltodextrine bzw. Polymaltosen, die durch enzymatische Spaltung von Mais- oder Kartoffelstärke mit alpha-Amylase hergestellt werden und die durch den Hydrolysegrad ausgedrückt durch den DE-Wert (Dextrose-Äquivalent) charakterisiert werden. Polymaltose kann erfindungsgemäß auch durch saure Hydrolyse von Dextrinen erhalten werden. Die Herstellung der erfindungsgemäß anwendbaren Eisen(III)-Komplexverbindungen erfolgt im allgemeinen durch Umsetzung von Eisen(II)- oder (III)-salzen, insbesondere Eisen(III)-chlorid, mit den Dextrinen, insbesondere Polymaltose, oder Oxidationsprodukten der Dextrine in wässriger alkalischer Lösung (pH > 7) und anschließender Aufarbeitung. Die Herstellung gelingt auch im schwach sauren pH-Bereich. Bevorzugt sind jedoch alkalische pH-Werte von beispielsweise >10.

Das Anheben des pH-Wertes erfolgt bevorzugt langsam bzw. allmählich, was beispielsweise dadurch erfolgen kann, dass zunächst eine schwache Base zugesetzt wird, beispielsweise bis zu einem pH von etwa 3; anschließend kann dann mit einer stärkeren Base weiter neutralisiert werden. Als schwache Base kommen beispielsweise Alkali- oder Erdalkalicarbonate, -bicarbonate, wie Natrium- und Kaliumcarbonat oder -bicarbonat oder Ammoniak infrage. Starke Basen sind beispielsweise Alkali- oder Erdalkalihydroxide, wie Natrium-, Kalium-, Calcium-oder Magnesiumhydroxid.

Die Umsetzung kann durch Erwärmen begünstigt werden. Beispielsweise können Temperaturen in der Größenordnung von 15°C bis zur Siedetemperatur angewendet werden. Es ist bevorzugt, die Temperatur allmählich zu steigern. So kann beispielsweise zunächst auf etwa 15 bis 70°C erwärmt und allmählich bis zum Sieden gesteigert werden.

Die Reaktionszeiten liegen beispielsweise in der Größenordnung von 15 Minuten bis zu mehreren Stunden, z.B. 20 Minuten bis 4 Stunden, beispielsweise bei 25 bis 70 Minuten, z. B. 30 bis 60 Minuten. Die Reaktionszeiten liegen beispielsweise in der Größenordnung von 15 Minuten bis zu mehreren Stunden, z.B. 20 Minuten bis 4 Stunden, beispielsweise bei 25 bis 70 Minuten, z. B. 30 bis 60 Minuten.

Nach erfolgter Umsetzung kann die erhaltene Lösung beispielsweise auf Raumtemperatur abgekühlt und gegebenenfalls verdünnt und gegebenenfalls filtriert werden. Nach dem Abkühlen kann der pH-Wert durch Zugabe von Säure oder Base auf den Neutralpunkt oder leicht darunter, beispielsweise auf Werte von 5 bis 7 eingestellt werden. Als Basen können beispielsweise die vorstehend zur Umsetzung genannten verwendet werden. Säuren schließen beispielsweise Salzsäure und Schwefelsäure ein. Die erhaltenen Lösungen werden gereinigt und können direkt zur Herstellung von Arzneimitteln verwendet werden. Es ist aber auch möglich, die Eisen(III)-Komplexe aus der Lösung zu isolieren, beispielsweise durch Ausfällen mit einem Alkohol, wie einem Alkanol, beispielsweise Ethanol. Die Isolierung kann auch durch Sprühtrocknung erfolgen. Die Reinigung kann in üblicher Weise erfolgen, insbesondere zur Entfernung von Salzen, Dies kann z. B. durch Umkehrosmose erfolgen, wobei eine derartige Umkehrosmose z. B. vor der Sprühtrocknung oder vor dem direkten Einsatz in Arzneimitteln durchgeführt werden kann.

Die erhaltenen Eisen(III)-Komplexe weisen beispielsweise einen Eisengehalt von 10 bis 40 % Gew./Gew., insbesondere 20 bis 35% Gew./Gew. auf. Sie sind im allgemeinen gut wasserlöslich. Man kann daraus neutrale wässrige Lösungen mit beispielsweise 1 % Gew./Vol. bis 20 % Gew./Vol. Eisengehalt herstellen. Diese Lösungen lassen sich thermisch sterilisieren.

Bezüglich der Herstellung von Eisen(III)-Polymaltose-Komplexverbindungen kann auch auf die US 3076798 verwiesen werden.

In einer bevorzugten Ausführungsform der Erfindung wird eine Eisen(III)-hydroxid-Polymaltose-Komplexverbindung verwendet. Bevorzugt besitzt diese die Eisen(III)-Polymaltose-Komplexverbindung ein Molekulargewicht im Bereich von 20000 bis 500000, in einer bevorzugten Ausführungsform 30000 bis 80000 Dalton (bestimmt mittels Gelpermeationschromatogrophie, beispielsweise wie von Geisser et al. In Arzneim. Forsch/Drug Res. 42(11), 12,1439-1452 (1992), Absatz 2.2. 5. beschrieben). Eine besonders bevorzugte Eisen(III)-hydroxid-Polymaltose-Komplexverbindung ist das im Handel erhältlich Maltofer® der Firma Vifor AG, Schweiz. In einer weiteren bevorzugten Ausführungsform wird eine Eisen(III)-Komplexverbindung mit einem Oxidationsprodukt von einem oder mehreren Maltodextrinen verwendet. Diese ist beispielweise erhältlich aus einer wässrigen Eisen(III)-Salzlösung und einer wässrigen Lösung des Produktes der Oxidation von einem oder mehreren Maltodextrinen mit einer wässrigen Hypochloritlösung bei einem pH-Wert im alkalischen Bereich, wobei beim Einsatz von einem Maltodextrin dessen Dextrose-Äquivalent bei 5 bis 37 und beim Einsatz eines Gemisches aus mehreren Maltodextrinen das Dextrose-Äquivalent des Gemisches bei 5 bis 37 und das Dextrose Äquivalent der am Gemisch beteiligten einzelnen Maltodextrine bei 2 bis 40 liegt. Das gewichtsmittlere Molekulargewicht Mw der so erhaltenen Komplexe beträgt beispielsweise 30 kDa bis 500 kDa, bevorzugt 80 bis 350 kDa, besonders bevorzugt bis zu 300 kDa (bestimmt mittels Gelpermeationschromatographie, beispielsweise wie von Geisser et al. In Arzneim. Forsch/Drug Res. 42(11), 12,1439-1452 (1992), Absatz 2.2. 5. beschrieben). Diesbezüglich kann beispielsweise auf die WO 2004037865 A1 verwiesen werden, deren Offenbarungsgehalt vollumfänglich in vorliegender Anmeldung eingeschlossen sein soll.

Bezüglich der Herstellung von Eisen-Komplexverbindungen mit hydrierten Dextrinen kann auf die WO 03/087164 verwiesen werden.

Bezüglich der Herstellung von Eisen(III)-Pullulan-Komplexverbindungen kann auf die WO 02/46241 verwiesen werden.

Die erfindungsgemäß verwendeten Eisen(III)-hydroxid-Komplexverbindungen werden bevorzugt oral verabreicht. Prinzipiell können sie aber auch parenteral, wie intravenös, aber auch intramuskulär verabreicht werden. Die orale tägliche Dosis beträgt beispielsweise zwischen 10 und 500 mg Eisen/Tag der Anwendung. Die Verabreichung kann bedenkenlos über einen Zeitraum von mehreren Monaten bis zur Verbesserung des Eisenstatus, reflektiert durch den Hämoglobin-Wert, die Transferrin-Sättigung und den Ferritin-Wert, der Patienten eingenommen werden. Die orale Verabreichung erfolgt bevorzugt in Form einer Tablette, einer Kapsel, einer wässrigen Lösung oder Emulsion, als Granulat, Kapsel, Gel oder als Sachet. Die Anwendung von Lösungen oder Emulsionen ist besonders bei Kindern in der Form von Sirups bzw. Säften, Tropfen etc. bevorzugt. Dazu können die Eisen(III)-hydroxid-Dextrin-Komplex-Verbindungen mit üblichen pharmazeutischen Träger- bzw. Hilfsstoffen in die geeignete Verabreichungsform gebracht werden. Dazu können übliche Bindemittel bzw. Gleitmittel, Verdünnungsmittel, Desintegrationsmittel etc. verwendet werden.

Die erfindungsgemäße Verwendung kann bei Kindern, Jugendlichen und Erwachsenen erfolgen. Sie erfolgt bevorzugt zur Herstellung eines Arzneimittels zur Behandlung von Kindern. Kinder schließen dabei Säuglinge und Heranwachsende bzw. Jugendliche ein.

Die erfindungsgemäße Verwendung verläuft insbesondere mittels Verbesserung der Neutrophilen-Niveaus, der Antikörperniveaus und/oder der Lymphozytenfunktion, bestimmt beispielsweise durch die Lymphozytenreaktion gegenüber Phytohämagalutinin.

Die erfindungsgemäße Verwendung dient zur Behandlung von

### Patienten ohne Eisenmangelanämie oder Eisenmangel.

Diese Einteilung hängt vom Alter und Geschlecht der Patienten ab und kann auch individuell variieren. Die Einteilung kann beispielsweise durch den Hämoglobinwert und den Wert für die Transferrinsättigung (%) erfolgen. Referenzwerte für Hämoglobin bestimmt durch Durchflußzytometrie (photometrisch): Cyanhämiglobinmethode sind beispielsweise gelistet bei Charite, Institut für Laboratoriumsmedizin und Pathobiochemie (http://www.charite.de/ilp/routlne/parameter.html). Die Transferrinsättigung ist bei Patienten ohne Eisenmangel in der Regel > 16 %.

Laut M. Wick, W, Pinggera, P. Lehmann, Eisenstoffwechsel - Diagnostik und Therapien der Anämien, 4., erw. Aufl. Springer Verlag Wien 1998 lassen sich alle Formen des Eisenmangels klinisch-chemisch nachweisen. Dabei geht im allgemeinen eine erniedrigte Ferritin-Konzentration mit kompensatorisch erhöhtem Transferrin und niedriger Transferrinsättigung einher.

Überraschend wurden erfindungsgemäß Verbesserungen der Immunabwehr und/oder der Hirnleistung bei Patienten erzielt, die über einen normalen hämatologische Befund hinsichtlich des Eisenstatus verfügten.

Die Erfindung wird in ihrer Wirkungsweise durch das folgende Beispiel erläutert und belegt.

### BEISPIEL

Unter Verwendung einer Filmtablette (620 mg je Tablette), die 357,0 mg einer Eisen(III)-hydroxid-Polymaltose-Komplexverbindung (Maltofer®) entsprechend 100 mg Eisen wurde eine Ein-Zentren-Studie über die orale Eisenergänzung bei Heranwachsenden mit Eisenmangel mit oder ohne Anämie gegenüber einer Placebogruppe durchgeführt.

Ziel der Vergleichsstudie war es, die Wirkungen des oral verabreichten Eisen(III)-Hydroxid-Polymaltose-Komplexes (100 mg Eisen pro Tag und Placebo während 6 Tagen pro Woche für 8 Monate) auf die Immunantworten, den hämatologischen Status, die kognitiven Funktionen und die Verhaltensfunktionen in vier Gruppen von Heranwachsenden mit Eisenmangelanämie, mit Eisenmangel, ohne Eisenmangel und Anämie (Eisen-Ergänzung) und einer weitere Gruppe ohne Eisenmangel und Anämie (Placebogruppe) zu untersuchen.

Dazu wurde 500 augenscheinlich gesunde Heranwachsende beider Geschlechter im Alter zwischen 15 und 18 Jahren zufällig ausgewählt und unter Verwendung mehrerer hämatologischer Kriterien in folgende Gruppen eingeteilt.

| Gruppe | Hämoglobin (g/dl) | Transferrinsättigung (%) |
|---|---|---|
| A: Eisenmangelanämie (IDA) (Vergleich) | Jungen: 7 - < 11,5 Mädchen: 7 - < 10,5 | < 16 < 16 |
| B: Eisenmangel (ID) (Vergleich) | Jungen: ≥ 11,5 Mädchen: ≥ 10,5 | < 16 < 16 |
| C: kein Eisenmangel, keine Anämie, Normal-Ergänzung (NS) | Jungen: ≥ 11,5 Mädchen: ≥ 10,5 | ≥ 16 ≥ 16 |
| D: Placebo keine Anämie, Normalplacebo (NP) | Jungen: ≥ 11,5 Mädchen: ≥ 10,5 | ≥ 16 ≥ 16 |

Jede Gruppe bestand aus 30 Testpersonen und es gab insgesamt 120 Testpersonen, die in die oben erwähnten vier Gruppen eingeteilt wurden (NP, NS, ID und IDA).

### Testverfahren:

### Hämatologische Bewertung:

Venöses Blut wurde allen 120 Testpersonen unter Verwendung von Vacuteströhrchen entnommen, und in einem Sysmax-Bluttest wurden die gesamten Blutparameter analysiert. Die Seren wurden aus den Blutproben abgetrennt und bei -20°C gelagert. Serenproben wurden SFe- und TIBC-Bestimmungen durch Standardverfahren (NIN-Manual 1983) unterworfen. TS wurde berechnet aus SFe und TIBC und als Prozentsatz angegeben. Serum-Ferritin-Analysen wurden unter Verwendung eines "Enzyme Linked Immuno Sorbent Assay" (ELISA) durchgeführt.

Serumfolat und Cyanacobalamin (B12) wurden durch Standardverfahren bestimmt.

### Immunantworten:

Aus frischen Blutproben wurden Neutrophile und Lymphozyten durch die Einstufen-Doppeldichtegradienten-Methode abgetrennt und die folgenden immunologischen Assays wurden für alle 120 Testpersonen durchgeführt:
Lymphozyten-Antwort gegenüber Phytohämagglutinin (PHA) unter Verwendung der MTT-Methode,
Nitroblau-Tetrazoliumtest (MBT) unter Verwendung von Neutrophilen,
Bakterizide Kapazität der Neutrophile (BCA) gemessen durch die turbidimetrische Methode.
Unter Verwendung von frischen Proben des gesamten Blutes wurden Zählungen der monoklonalen Antikörper CD3, CD4, CD8 und CD56 unter Verwendung eines BD-Fließzytometers durch die Einzelfärbungsmethode durchgeführt und als Prozentsatz für alle 120 Subjekte ausgedrückt.
Unter Verwendung von Serenproben aller 120 Testpersonen wurden die folgenden Tests durchgeführt:
   Antikörperantworten gegenüber Masern, Grippeviren und Tetanus.
   C-Reaktives Protein unter Verwendung einer halbquantitativen Methode.

### Kognitive Funktionen und emotionales Verhalten

Die kognitiven Funktionen und das emotionale Verhalten der 120 Testpersonen wurden gemäß den am häufigsten verwendeten Tests und gemäß etablierter Methodik bestimmt:
1. Kurzzeitgedächtnistest (STM)
2. Langzeitgedächtnistest (LTM)
3. Progressive Matrices gemäß Raven (RPM)
4. Weschlers Erwachsenenintelligenzskala (WAIS)
5. Emotionaler Quotient (Baron EQ-i; YV-Test; Jugendversion)

### Schulleistungsbewertung

Die Schulleistungen der 120 Testpersonen wurden individuell durch einen Schulleistungstest bestimmt.

Anthropometrische Bewertungen, physische Untersuchungen, Urin- und Stuhluntersuchungen wurden nach Standardverfahren durchgeführt.

### Statistische Auswertung:

Der Vergleich der Durchschnittswerte der Parameter über aufeinanderfolgende Zeitperioden ergab ein Bild der Wirkung der Behandlung. Naturbedingt erforderte die Auswertung ein "repeated measures design". Für jeden Parameter gab es drei Sätze von Beobachtungen, die alle korreliert waren. Folglich ist der "repeated measures-Test" das geeignete Mittel, und die statistische Analyse wurde mit Hilfe der SPSS-Software durchgeführt. Die Methode testet die Signifikanz der Parameterwerte über die Dauer (Zeitpunkte) zwischen den Gruppen und, falls vorhanden, Interaktion zwischen Dauer und Gruppe. Mehrfachvergleiche zwischen verschiedenen Gruppen wurden automatisch durch den "repeated measures-Test" mit Hilfe der LSD (Least Significance Difference Test) durchgeführt.

### Ergebnisse

Günstige Wirkungen wurden bei allen durchgeführten Tests gefunden, nämlich hinsichtlich des hämatologischen Status, des Immunstatus der kognitiven Funktion und des emotionalen Verhaltens.

Signifikante Inkremente wurden für alle eisenverbundenen hämatologischen Parameter von der Basislinie bis vier Monate und erneut von vier Monate bis acht Monate der Ergänzung in allen Gruppen, denen das Eisenpräparat gegeben wurde, nämlich der Gruppe mit Eisenmangel mit (IDA) (Vergleich) oder ohne Anämie (ID) (Vergleich) und der mit normaler Hämatologie, die ergänzt wurde (NS) (erfindungsgemäß) gefunden.

Es gab einen signifikanten Anstieg in der Lymphozytenantwort gegenüber Phytohämagglutinin, Bakterienabtötung durch Neutrophil-Assay, Nitroblau-Tetrazoliumtest und der Niveaus der Antikörper gegenüber Masern und Hämophilus influenza in den drei ergänzenden Gruppen (IDA, ID, NS) für die Zeiträume 0 bis 4 Monate und 4 bis 8 Monate. Für Antikörper gegenüber Tetanus war der Unterschied zwischen Basislinie und 8 Monaten signifikant für diese drei Gruppen.

Die kognitive Leistung verbesserte sich von der Basislinie bis vier Monate und von 4 bis 8 Monate bei allen Tests, einschließlich SAT, ausgenommen EQ.

Diese Inkremente in hämatologischen, immunologischen und kognitiven Funktionen besitzen eine bedeutende klinische Signifikanz. Durch die regelmäßige Verabreichung eines Eisen(III)-Hydroxid-Polymaltose-Komplexes kann die Immunabwehr und die Hirnleistung verbessert werden mit enormen Vorteilen für die Gesellschaft.

## Patentansprüche

1. Verwendung von Eisen(III)-Komplexverbindungen mit Kohlenhydraten, zur Herstellung eines Arzneimittels zur Verbesserung der Immunabwehr und/oder der Hirnleistung bei Patienten ohne Eisenmangelanämie oder Eisenmangel, worin die Eisen(III)-Komplexverbindung eine Eisen(III)-Polymaltose-Komplexverbindung oder eine Eisen(III)-Komplexverbindung mit einem Oxidationsprodukt von einem oder mehreren Maltodextrinen ist.

2. Verwendung nach Anspruch 1, worin die Eisen(III)-Polymaltose-Komplexverbindung ein Molekulargewicht im Bereich von 20000 bis 500000 Dalton besitzt.

3. Verwendung nach Anspruch 1, worin die Eisen(III)-Komplexverbindung mit einem Oxidationsprodukt von einem oder mehreren Maltodextrinen ein wasserlöslicher Eisen-Kohlenhydrat-Komplex ist, erhältlich aus einer wässrigen Eisen(III)-Salzlösung und einer wässrigen Lösung des Produktes der Oxidation von einem oder mehreren Maltodextrinen mit einer wässrigen Hypochloritlösung bei einem pH-Wert im alkalischen Bereich, wobei beim Einsatz von einem Maltodextrin dessen Dextrose-Äquivalent bei 5 bis 37 und beim Einsatz eines Gemisches aus mehreren Maltodextrinen das Dextrose-Äquivalent des Gemisches bei 5 bis 37 und das Dextrose Äquivalent der am Gemisch beteiligten einzelnen Maltodextrine bei 2 bis 40 liegt.

4. Verwendung nach einem der Ansprüche 1 bis 3 zur Herstellung eines Arzneimittels zur oralen oder parenteralen Verabreichung.

5. Verwendung nach einem der Ansprüche 1 bis 4 zur Herstellung eines Arzneimittels zur Behandlung von Kindern, Jugendlichen oder Erwachsenen.

6. Verwendung nach einem der Ansprüche 1 bis 5, worin das Arzneimittel in der Form einer Tablette, einer wässrigen Lösung oder Emulsion, als Granulat, Kapsel, Gel oder als Sachet vorliegt.

7. Verwendung nach einem der Ansprüche 1 bis 6 zur Verbesserung der Neutrophilen-Niveaus, der Antikörperniveaus und/oder der Lymphozytenfunktion, bestimmt durch die Lymphozytenreaktion gegenüber Phytohämagglutinin.

## Claims

1. Use of iron(III)-polymaltose complex compounds or iron(III) complex compounds with an oxidation product of one or more maltodextrins in the preparation of a medicament for improving immune defence and/or brain performance in patients without iron deficiency anaemia or iron deficiency.

2. Use according to claim 1, wherein the iron(III)-polymaltose complex compound has a molecular weight in the range from 20,000 to 500,000 daltons.

3. Use according to claim 1, wherein the iron(III) complex compound with an oxidation product of one or more maltodextrins is a watersoluble iron-carbohydrate complex obtainable from an aqueous iron(III) salt solution and an aqueous solution of the product of the oxidation of one or more maltodextrins with an aqueous hypochlorite solution at a pH value in the alkaline range, wherein when a maltodextrin whose dextrose equivalent is from 5 to 37 is used and when a mixture of a plurality of maltodextrins is used, the dextrose equivalent of the mixture is from 5 to 37 and the dextrose equivalent of the individual maltodextrins contained in the mixture is from 2 to 40.

4. Use according to any one of claims 1 to 3 in the preparation of a medicament for oral or parenteral administration.

5. Use according to any one of claims 1 to 4 in the preparation of a medicament for treating children, young people or adults.

6. Use according to any one of claims 1 to 5, wherein the medicament is present in the form of a tablet, of an aqueous solution or emulsion, in the form of granules, a capsule, a gel or in the form of a sachet.

7. Use according to any one of claims 1 to 6 for improving the neutrophil level, the antibody level and/or the lymphocyte function, determined by the lymphocyte reaction to phytohaemagglutinin.

## Revendications

1. Utilisation de composés complexes de fer(III)-polymaltose ou du composé complexe de fer(III) avec un produit d'oxydation d'une ou plusieurs maltodextrines pour la préparation d'un médicament destiné à améliorer la défense immunitaire et/ou la capacité cérébrale chez des patients exempts d'une anémie ferriprive ou d'une sidéropénie.

2. Utilisation selon la revendication 1, dans laquelle le composé de complexe de fer(III)-polymaltose possède un poids moléculaire dans la plage de 20.000 à 500.000 Dalton.

3. Utilisation selon la revendication 1, dans laquelle le composé complexe de fer(III) avec un produit d'oxydation d'une ou plusieurs maltodextrines et un complexe de fer-hydrate de carbone soluble dans l'eau, que l'on obtient à partir d'une solution saline aqueuse de fer(III) et d'une solution aqueuse du produit de l'oxydation d'une ou de plusieurs maltodextrines avec une solution aqueuse d'hypochlorite à une valeur de pH dans la plage alcaline, dans laquelle, lorsqu'on met en oeuvre une maltodextrine dont l'équivalent en dextrose s'élève de 5 à 37 et lorsqu'on met en oeuvre un mélange de plusieurs maltodextrines, l'équivalent en dextrose du mélange s'élève de 5 à 37 et l'équivalent en dextrose des maltodextrines individuelles intervenant dans le mélange s'élève de 2 à 40.

4. Utilisation selon l'une quelconque des revendications 1 à 3, pour la préparation d'un médicament destiné à une administration par voie orale ou par voie parentérale.

5. Utilisation selon l'une quelconque des revendications 1 à 4, pour la préparation d'un médicament destiné au traitement des enfants, des adolescents ou des adultes.

6. Utilisation selon l'une quelconque des revendications 1 à 5, dans laquelle le médicament se présente sous la forme d'un comprimé, d'une solution ou d'une émulsion aqueuse, sous la forme d'un produit de granulation, d'une capsule, d'un gel ou sous la forme d'un sachet.

7. Utilisation selon l'une quelconque des revendications 1 à 6, pour améliorer le tout ou des polynucléaires neutrophiles, le taux des anticorps et/ou les fonctions lymphocytaires, déterminé par la réaction des lymphocytes vis-à-vis de la phytohémagglutinine.
